(19) Europäisches Patentamt
European Patent Office
Office européen des brevets

(11) EP 1 861 354 B1

# (12) EUROPÄISCHE PATENTSCHRIFT

(45) Veröffentlichungstag und Bekanntmachung des Hinweises auf die Patenterteilung:
**23.07.2008 Patentblatt 2008/30**

(21) Anmeldenummer: **06708713.0**

(22) Anmeldetag: **10.03.2006**

(51) Int Cl.:
***C07C 69/24*** *(2006.01)* ***C11D 1/74*** *(2006.01)*
***C11D 3/00*** *(2006.01)*

(86) Internationale Anmeldenummer:
**PCT/EP2006/060613**

(87) Internationale Veröffentlichungsnummer:
**WO 2006/097435 (21.09.2006 Gazette 2006/38)**

## (54) VERESTERTE ALKYLALKOXYLATE ALS SCHAUMARME TENSIDE

ESTERIFIED ALKYL ALKOXYLATES USED AS LOW-FOAM SURFACTANTS

ALKYLALKOXYLATES ESTÉRIFIÉS SERVANT DE TENSIOSACTIFS PEU MOUSSANTS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.03.2005 DE 102005011608**

(43) Veröffentlichungstag der Anmeldung:
**05.12.2007 Patentblatt 2007/49**

(73) Patentinhaber: **BASF SE**
**67056 Ludwigshafen (DE)**

(72) Erfinder:
- **BITTNER, Christian**
  **68305 Mannheim (DE)**
- **TROPSCH, Jürgen**
  **67354 Römerberg (DE)**
- **NÖRENBERG, Ralf**
  **67063 Ludwigshafen (DE)**

(74) Vertreter: **Huhn, Michael**
**Isenbruck Bösl Hörschler Wichmann Huhn Patentanwälte**
**Theodor-Heuss-Anlage 12**
**68165 Mannheim (DE)**

(56) Entgegenhaltungen:
**WO-A-94/03251** **DE-B- 1 243 312**

- **SZYMANOWSKI, J. ET AL: "Synthesis and properties of esterification products of some oxyethylated alcohols and alkylphenols with fatty acids" FETTE, SEIFEN, ANSTRICHMITTEL, Bd. 82, Nr. 6, 1980, Seiten 244-249, XP009069819**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 861 354 B1

## Beschreibung

Die vorliegende Erfindung betrifft schaumarme Tensid-Mischungen, sowie deren Herstellung. Die Tenside eignen sich für Wasch- und Reinigungsmittelformulierungen.

Tenside sind Substanzen, die die Grenzflächenspannung herabsetzen können. Typischerweise verfügen Tenside über einen charakteristischen Aufbau und weisen mindestens eine hydrophile und mindestens eine hydrophobe funktionelle Gruppe auf. Wenn beide Teile des Moleküls im Gleichgewicht zueinander stehen, wird sich die Substanz an einer Grenzfläche anreichern und ausrichten, d.h. hydrophile Gruppen weisen beispielsweise in eine wässrige Phase und die hydrophoben Gruppen in Richtung anderer fester, flüssiger oder gasförmiger Phasen. Eine weitere Besonderheit von Tensiden ist die Bildung höherer Aggregate, den sogenannten Micellen. Bei diesen ordnen sich die Tensidmoleküle dergestalt an, dass die polaren Gruppen zum Beispiel eine Kugelschale bilden. Dies bewirkt, dass Stoffe wie Schmutzpartikel in einer wässrigen Lösung unter Ausbildung von Micellen löslich gemacht werden.

Daher eignen sich Tenside insbesondere zur Reinigung von Oberflächen und als Zusatz in Waschmitteln.

Tenside, die einen hydrophoben und einen hydrophilen Bauteil aufweisen, sind weit verbreitet. Ihre Neigung zur Schaumbildung macht sie aber für viele Anwendungen nicht oder nur bedingt brauchbar. Daher wurden insbesondere nicht-ionische Tenside vorgeschlagen, die einen zweiten hydrophoben Block aufweisen, so dass das Schaumvolumen begrenzt wird.

DE-A 12 43 312 beschreibt beispielsweise die Verwendung von Alkylalkoxylaten, welche mit einer aliphatischen kurzkettigen oder aromatischen Carbonsäure verestert sind, als schaumarme nicht-ionische Tenside.

Ähnliche Verbindungen werden in DE-A 25 44 707 offenbart. Auch hier wird die Säurekomponente durch eine kurzkettige aliphatische Säure, nämlich Essigsäure, gebildet.

EP-A 035 702 offenbart Schaumdämpfungsmittel, die nicht-ionische Tenside beinhalten. Diese Tenside sollen 3 bis 10 Ethylenoxideinheiten beinhalten.

WO-A 94/03251 offenbart endgruppenverschlossene Antischaummittel, bei denen als Alkoholkomponente ein Fettalkoholpolyglykolether eingesetzt wird, der ebenfalls vorzugsweise bis 10 Ethylen- oder Propylenoxideinheiten enthält.

Den bekannten nicht-ionischen Tensiden ist gemein, dass deren schaumdämpfende Eigenschaften mit nur moderaten Wasch- und Reinigungseigenschaften einhergehen. Insbesondere kann die Formulierbarkeit im Alkalischen bei niederkettigen Tensiden problematisch sein.

Die Aufgabe der vorliegenden Erfindung ist daher, schaumarme, insbesondere feste, Tenside bereitzustellen, deren Schaumbildung zumindest vergleichbar zu der bekannter Tenside ist und eine verbesserte Wasch- und/oder Reinigungskraft aufweisen.

Die Aufgabe wird gelöst durch eine schaumarme Tensid-Mischung enthaltend Verbindungen der allgemeinen Formel (I)

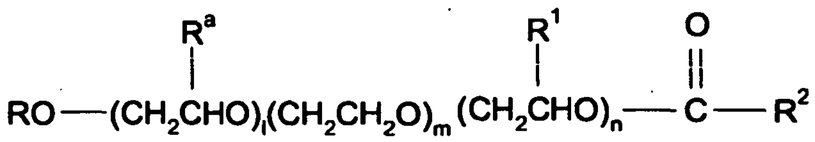

(I)

wobei

| | |
|---|---|
| R | ein verzweigter oder unverzweigter Alkylrest mit 8 bis 16 Kohlenstoffatomen ist; |
| $R^a$, $R^1$ | unabhängig voneinander Wasserstoff oder ein verzweigter oder unverzweigter Alkylrest mit 1 bis 5 Kohlenstoffatomen sind; |
| $R^2$ | ein unverzweigter Alkylrest mit 5 bis 17 Kohlenstoffatomen ist; |
| l, n | unabhängig voneinander eine Zahl von 1 bis 5 sind und |
| m | eine Zahl von 13 bis 35 ist. |

Es wurde nämlich gefunden, dass die Tensid-Mischungen trotz ihres hohen HLB-Wertes eine überraschenderweise hervorragende Schaumdämpfung bei gutem Netzvermögen vorzugsweise in einem Temperaturbereich von 0 bis 120 °C aufweisen können.

Der HLB-Wert ergibt sich dabei als Quotient aus Menge an Ethylenoxid zu Gesamtmenge x 20. Allgemein ist der HLB-Wert definiert durch die Formel

$$HLB = 20\left(1 - \frac{M_L}{M_G}\right),$$

wobei $M_L$ das Molekulargewicht der lipophilen Anteile und $M_G$ das Gesamtgewicht angibt. Nähere Einzelheiten hierzu finden sich in H.-D. Dörfer, Grenzflächen und kolloiddisperse Systeme, Springer Verlag 2002, Kapitel 9.3 "Physikalische Eigenschaften und Wirkungen der Tenside"

Es wurde weiterhin gefunden, dass die erfindungsgemäßen Tensid-Mischungen auch im alkalischen Anwendungsbereich stabil sein können. Dies macht ihre Formulierbarkeit in alkalischen Reinigern möglich.

Weiterhin zeigt sich, dass die erfindungsgemäßen Tensid-Mischungen bei Raumtemperatur in festem Zustand vorliegen können. Hierdurch lassen sich diese leichter in festen Waschmittel- oder Reinigungsmittelformulierungen einsetzen.

Ebenso zeigt sich, dass die erfindungsgemäßen Tenside, insbesondere wenn die Glykoleinheit, mit der die Säurekomponente verestert ist, eine andere ais Ethylenglykol ist, langzeitstabil sein können. Dies ist besonders vorteilhaft für Vorgänge die eine gewisse Mindestzeit, wie etwa beim Waschvorgang, benötigen.

Bevorzugte Tensid-Mischungen gemäß der vorliegenden Erfindung enthalten Verbindungen der allgemeinen Formel (II)

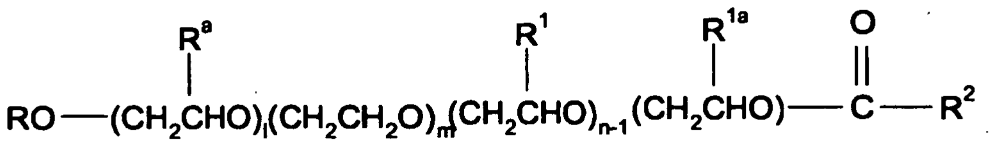

(II)

wobei $R^{1a}$ ein verzweigter oder unverzweigter Alkylrest mit 1 bis 5 Kohlenstoffatomen ist und R, $R^a$, $R^1$, $R^2$, l, m und n die obige Bedeutung haben.

Der Ausdruck "Alkylrest" bedeutet im Rahmen der vorliegenden Erfindung einen gesättigten verzweigten oder unverzweigten aliphatischen Kohlenwasserstoffrest mit der jeweils angegebenen Zahl an Kohlenstoffatomen.

Weiterhin können die Tensid-Mischungen der vorliegenden Erfindung zusätzlich Verbindungen der Formel (III) enthalten.

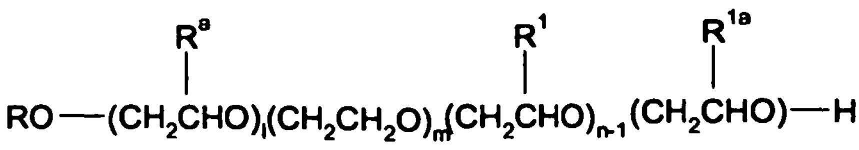

(III)

In diesem Fall liegt der Polyalkohol in seiner unveresterten Form vor. Vorzugsweise beträgt jedoch das molare Verhältnis Ester (I) zu Alkohol (III) mindestens 1 : 1, mehr bevorzugt mindestens 2 : 1 und am meisten bevorzugt mindestens 3 : 1.

Es ist weiterhin bevorzugt, dass mindestens 50%, vorzugsweise mindestens 75% und mehr bevorzugt mindestens 90% der Verbindungen in der erfindungsgemäßen Tensid-Mischung Verbindungen der Formel (I), (II) oder (III) sind.

Vorzugsweise ist der Rest R ein verzweigter Alkylrest mit 9 bis 16, mehr bevorzugt mit 10 bis 13, Kohlenstoffatomen. Der Verzweigungsgrad beträgt bevorzugt 1-3. Unter dem Begriff "Verzweigungsgrad" ist im Rahmen der vorliegenden Erfindung die um eins verminderte Anzahl an Methylgruppen zu verstehen.

Weiterhin bevorzugt sind $R^a$, $R^1$ unabhängig voneinander Wasserstoff, Methyl und Ethyl. Treten $R^a$, $R^1$ häufiger auf, so kann jedes unabhängig von einem weiteren $R^a$ oder $R^1$ ausgewählt werden. So können $R^a$, $R^1$ blockweise oder statistisch verteilt auftreten.

$R^{1a}$ ist vorzugsweise Methyl oder Ethyl.

$R^2$ ist bevorzugt ein verzweigter oder unverzweigter Alkylrest mit 5 bis 13 Kohlenstoffatomen.

Vorzugsweise ist n = 1, l = 5 und m bevorzugt eine Zahl von 13 bis 34, mehr bevorzugt 13 bis 33, weiterhin mehr bevorzugt 13 bis 30, am meisten bevorzugt 17 bis 27.

Weiterhin bevorzugt liegt das mittlere Molekulargewicht in einem Bereich von 950 bis 2300 g/mol. Besonders

bevorzugt liegt das mittlere Molekulargewicht in einem Bereich von 1200 bis 1900 g/mol.

Vorzugsweise sind mehr als 50% der Verbindungen der Tensid-Mischung nach der vorliegenden Erfindung Verbindungen der Formel (II) oder Verbindungen der Formel (I), wobei in den Verbindungen der Formel (I) mindestens ein $R^1$ kein Wasserstoff ist.

Vorzugsweise weist die Tensid-Mischung einen Beginn des Schmelzbereiches auf, der über 25 °C liegt. Mehr bevorzugt liegt der Wert über 30°C.

Ein weiterer Gegenstand der vorliegenden Erfindung ist die Herstellung von Tensid-Mischungen die Schritte enthaltend:

a) Umsetzung eines Alkohols der Formel ROH mit einem Epoxid der Formel

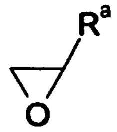

und anschließend mit Ethylenoxid;

b) Umsetzung des Produktes aus Schritt a) mit einem Epoxid der Formel

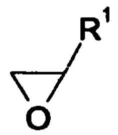

und optional mit einem Epoxid der Formel

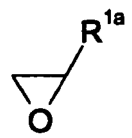

c) Umsetzung des gebildeten Produkts aus Schritt b) mit einer Carbonsäure $R^2$-COOH oder eines Methylesters $R^2$-$COOCH_3$, wobei $R^1$, $R^{1a}$ und $R^2$ die Bedeutung wie in Anspruch 1 oder 2 aufweisen.

Sofern in Schritt a) $R^a$ = H ist, erfolgt die Umsetzung nur mit Ethylenoxid.

Vorzugsweise erfolgen die Schritte a) und b) durch wasserfreie basenkatalysierte Umsetzung. Hierbei wird als Base vorzugsweise Natriumhydroxid oder Kaliumhydroxid eingesetzt. Der Temperaturbereich beträgt vorzugsweise 50 bis 200 °C.

Die Umsetzung in Schritt c) erfolgt vorzugsweise säure- oder basekatalysiert; als Säure wird vorzugsweise Schwefelsäure oder Paratoluolsulfonsäure eingesetzt. Der Temperaturbereich kann bei Schritt c) von 80 bis 200 °C betragen. Vorzugsweise findet die Umsetzung in Schritt c) unter kontinuierlicher Entfernung des Reaktionswassers bzw. Methanols statt. Dies erfolgt beispielsweise bei Normaldruck und/oder Strippen mit Stickstoff oder reduziertem Druck oder durch Verwendung eines Schleppmittels wie zum Beispiel Toluol oder Xylol im Falle des Wassers.

Die erfindungsgemäßen Tensid-Mischungen eignen sich besonders in Wasch- und Reinigungsmittelformulierungen. Daher ist ein weiterer Gegenstand der Erfindung eine Wasch- oder Reinigungsmittelformulierung enthaltend eine erfindungsgemäße Tensid-Mischung.

Besonders bevorzugt finden die Tensid-Mischungen in so genannten "2 in 1" oder "3 in 1" Tabs Verwendung. Näheres zu diesen Formulierungen findet sich in Hermann G. Hauthal, G. Wagner (Hrsg), Reinigungs- und Pflegemittel im Haushalt, Verlag für chemische Industrie, H. Ziolkowsky GmbH, Augsburg 2003, Kapitel 4.2, Seiten 161-184.

Waschmittel im Sinne dieser Erfindung dienen in der Regel zum Waschen von mehr oder weniger flexiblen Materialien, vorzugsweise solchen, die natürliche, synthetische oder halbsynthetische Fasermaterialien enthalten oder

daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen Lodenstoffen oder Filz, Geweben, Gewirken in allen denkbaren Bindungsarten vorliegen.

Es kann sich um Rohfasern oder um Fasern in beliebigen Verarbeitungsstadien handeln und es können natürliche Eiweiß- oder Zellulosefasern wie Wolle, Seide, Baumwolle, Sisal, Hanf, Kokosfasern oder Synthesefasern wie beispielsweise Polyester-, Polyamid- oder Polyacrylnitrilfasem sein.

Die erfindungsgemäßen Waschmittel können auch mit besonderem Vorteil im Rahmen der Verarbeitung von Fasermaterialien eingesetzt werden z.B. zum Entfetten von Rohwolle oder zum Entschlichten von Fasermaterialien aller Art.

Die erfindungsgemäßen Waschmittel können auch zur Reinigung von faserhaltigen Materialien, wie z.B. rükkenbeschichteten Teppichen mit geschnittenem oder ungeschnittenem Flor dienen.

Das erfindungsgemäße Reinigungsmittel eignet sich besonders gut zur Reinigung von Materialien mit geschlossener, insbesondere harter Oberfläche, d.h. von Oberflächen, die keine oder nur wenige und kleine Poren haben und infolgedessen keine oder nur eine geringe Saugfähigkeit aufweisen. Materialien mit geschlossenen Oberflächen sind überwiegend hart, können aber auch weich sein in dem Sinne, dass sie eine gewisse reversible oder irreversible Verformbarkeit aufweisen.

Beispiele für Materialien mit harten Oberflächen, für deren Reinigung die erfindungsgemäßen Reinigungsmittel vorzugsweise eingesetzt werden, sind Metall, Glas, Email, Keramik. Typische Objekte aus diesen Materialien sind z.B. metallene Spülbecken, Bestecke, Glas- und Porzellangeschirr, Badewannen, Waschbecken, Kacheln, Fliesen und ausgehärtete Kunstharze, wie z.B. dekorative Melaminharzoberflächen auf Küchenmöbeln oder lackierte Metallflächen wie z.B. Kühlschränke und Autokarosserien. Die erfindungsgemäßen Reinigungsmittel sind auch sehr wertvolle Hilfsmittel bei der Herstellung von gedruckten Schaltungen, wobei es darum geht, Fettspuren und andere Verunreinigungen von kupfer- oder silberkaschierten Trägem vor dem Ätzen und/oder vor der Bestückung zu entfernen und/oder nach der Bestückung Lötfette oder andere Flussmittelreste gründlich zu beseitigen.

Auch bei der Herstellung von Mikrochips können die erfindungsgemäßen Reinigungsmittel wertvolle Dienste leisten. Materialien mit geschlossenen, insbesondere harten Oberflächen im Sinne dieser Erfindung können auch zerklüftete Oberflächen aufweisen, wie sie beispielsweise bei Metallkeramik-Werkstoffen zu finden sind.

Beispiele für weichere Materialien, die mit der erfindungsgemäßen Reinigungsmittel gereinigt werden können, sind beispielsweise versiegelte oder lackierte Hölzer, z.B. Parkett oder Wandverkleidungen, Fensterrahmen, Türen, Kunststoffbeläge wie Fußbodenbeläge aus PVC oder Hartgummi, oder Hart- oder Weichschaumstoffe mit weitgehend geschlossenen Oberflächen.

Insbesondere können die erfindungsgemäßen Reiniger beispielsweise als Handgeschirrreiniger, Maschinengeschirrreiniger, Metallentfetter, Glasreiniger, Fußbodenreiniger, Allzweckreiniger, Hochdruckreiniger, Neutralreiniger, alkalische Reiniger, saure Reiniger, Spritzentfetter, Molkereireiniger, Großküchenreiniger, Apparatereiniger in der Industrie, insbesondere der chemischen Industrie, als Reiniger bei der Autowäsche aber auch als Haushalts-Allzweckreiniger eingesetzt werden.

Selbstverständlich werden die Zusammensetzungen der Wasch- und Reinigungsmittel den verschiedenen Zwecken angepasst, wie es dem Fachmann aus dem Stand der Technik geläufig ist. Hierzu können den erfindungsgemäßen Wasch- und Reinigungsmitteln alle zweckentsprechenden, aus dem oben genannten Stand der Technik bekannten Hilfs- und Zusatzstoffe zugefügt werden.

In vielen Fällen ist es zweckmäßig, die erfindungsgemäß eingesetzten Tensid-Mischungen der Formel (I) mit anderen nichtionischen Tensiden, wie z.B. Alkoholalkoxilaten, Alkylaminalkoxilaten, Alkylamidalkoxilaten, Alkylpolyglucosiden, oder mit ionischen, vorzugsweise anionischen, Tensiden, wie zum Beispiel längerkettigen oder langkettigen Alkoholsulfat/-ethersulfaten, Alkylbenzolsulfonaten, $\alpha$-Olefinsulfonaten, Sulfosuccinaten, oder mit amphoteren Tensiden, wie z.B. Alkylaminoxiden, oder Betainen zu kombinieren.

Im Folgenden werden Beispiele für zur Kombination geeignete Tenside unterschiedlicher Natur genannt:

Als nichtionische Tenside eignen sich beispielsweise alkoxylierte $C_8$- bis $C_{22}$-Alkohole wie Fettalkoholalkoxylate oder Oxoalkoholalkoxylate. Die Alkoxylierung kann mit Ethylenoxid, Propylenoxid und/oder Butylenoxid durchgeführt werden. Als Tenside einsetzbar sind hierbei sämtliche alkoxylierten Alkohole, die vorzugsweise mindestens zwei Moleküle eines vorstehend genannten Alkylenoxids addiert enthalten. Auch hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Pro Mol Alkohol verwendet man 2 bis 50, vorzugsweise 3 bis 20 Mol mindestens eines Alkylenoxids. Vorzugsweise setzt man als Alkylenoxid Ethylenoxid ein. Die Alkohole haben vorzugsweise 10 bis 18 Kohlenstoffatome. Je nach Art des Alkoxylierungskatalysators kann man Alkoxylate mit breiter oder enger Alkylenoxid-Homologen-Verteilung erhalten.

Eine weitere Klasse geeigneter nichtionischer Tenside sind Alkylphenolalkoxylate wie Alkylphenolethoxylate mit $C_6$ bis $C_{14}$-Alkylketten und 5 bis 30 Mol Alkylenoxideinheiten.

Eine andere Klasse nichtionischer Tenside sind Alkylpolyglucoside mit 6 bis 22, vorzugsweise 8 bis 18 Kohlenstoffatomen in der Alkylkette. Diese Verbindungen enthalten meist 1 bis 20, vorzugsweise 1,1 bis 5 Glucosideinheiten.

Eine andere Klasse nichtionischer Tenside sind N-Alkylglucamide der allgemeinen Strukturen

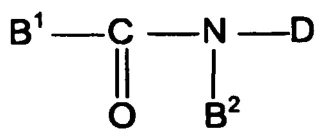 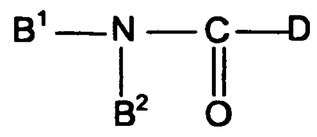

wobei $B^1$ ein $C_6$- bis $C_{22}$-Alkyl, $B^2$ Wasserstoff oder $C_1$- bis $C_4$-Alkyl und D ein Polyhydroxyalkyl-Rest mit 5 bis 12 C-Atomen und mindestens 3 Hydroxygruppen ist. Vorzugsweise steht $B^1$ für $C_{10}$- bis $C_{18}$-Alkyl, $B^2$ für $CH_3$ und D für einen $C_5$- oder $C_6$-Rest. Beispielsweise erhält man derartige Verbindungen durch die Acylierung von reduzierend aminierten Zuckern mit Säurechloriden von $C_{10}$- bis $C_{18}$-Carbonsäuren.

Weitere in Betracht kommende nichtionische Tenside sind die aus der WO-A 95/11225 bekannten endgruppenverschlossenen Fettsäureamidalkoxylate der allgemeinen Formel

$$R^1\text{-CO-NH-}(CH_2)_y\text{-O-}(A^1O)_x\text{-}R^2$$

in der

- $R^1$ einen $C_5$- bis $C_{21}$-Alkyl- oder Alkenylrest bezeichnet,
- $R^2$ eine $C_1$- bis $C_4$-Alkylgruppe bedeutet,
- $A^1$ für $C_2$- bis $C_4$-Alkylen steht,
- y die Zahl 2 oder 3 bezeichnet und
- x einen Wert von 1 bis 6 hat.

Beispiele für solche Verbindungen sind die Umsetzungsprodukte von n-Butyltriglykolamin der Formel $H_2N\text{-}(CH_2\text{-}CH_2\text{-}O)_3\text{-}C_4H_9$ mit Dodecansäuremethylester oder die Reaktionsprodukte von Ethyltetraglykolamin der Formel $H_2N\text{-}(CH_2\text{-}CH_2\text{-}O)_4\text{-}C_2H_5$ mit einem handelsüblichen Gemisch von gesättigten $C_8$- bis $C_{18}$-Fettsäuremethylestern.

Weiterhin eignen sich als nichtionische Tenside noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.

Die zusätzlichen nichtionischen Tenside("Niotenside") liegen in den erfindungsgemäßen Wasch- und Reinigungsmitteln vorzugsweise in einer Menge von 0,01 bis 30 Gew.-%, insbesondere 0,1 bis 25 Gew.-%, vor allem 0,5 bis 20 Gew.-%, vor.

Man kann einzelne nichtionische Tenside oder eine Kombination unterschiedlicher Niotenside einsetzen. Es können nichtionische Tenside aus nur einer Klasse zum Einsatz gelangen, insbesondere nur alkoxylierte $C_8$- bis $C_{22}$-Alkohole, man kann aber auch Tensid-Mischungen aus verschiedenen Klassen verwenden.

Geeignete anionische Tenside sind beispielsweise Fettalkoholsulfate von Fettalkoholen mit 8 bis 22, vorzugsweise 10 bis 18 Kohlenstoffatomen, z.B. $C_9$-$C_{11}$-Alkoholsulfate, $C_{12}$-$C_{14}$-Alkoholsulfate, $C_{12}$-$C_{18}$-Alkoholsulfate, Laurylsulfat, Cetylsulfat, Myristylsulfat, Palmitylsulfat, Stearylsulfat und Talgfettalkoholsulfat.

Weitere geeignete anionische Tenside sind sulfatierte ethoxylierte $C_8$-$C_{22}$-Alkohole (Alkylethersulfate) bzw. deren lösliche Salze. Verbindungen dieser Art werden beispielsweise dadurch hergestellt, dass man zunächst einen $C_8$- bis $C_{22}$-, vorzugsweise einen $C_{10}$-$C_{18}$-Alkohol z.B. einen Fettalkohol, alkoxyliert und das Alkoxylierungsprodukt anschließend sulfatiert. Für die Alkoxylierung verwendet man vorzugsweise Ethylenoxid, wobei man pro Mol Alkohol 1 bis 50, vorzugsweise 1 bis 20 Mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte $C_8$-$C_{22}$-Alkohole, die Ethylenoxid und Propylenoxid oder Ethylenoxid und Butylenoxid oder Ethylenoxid und Propylenoxid und Butylenoxid enthalten. Die alkoxylierten $C_8$-$C_{22}$-Alkohole können die Ethylenoxid-, Propylenoxid- und Butylenoxideinheiten in Form von Blöcken oder in statistischer Verteilung enthalten. Je nach Art des Alkoxylierungskatalysators kann man Alkylethersulfate mit breiter oder enger Alkylenoxid-Homologen-Verteilung erhalten.

Weitere geeignete anionische Tenside sind Alkansulfonate wie $C_8$-$C_{24}$-, vorzugsweise $C_{10}$-$C_{18}$-Alkansulfonate sowie Seifen wie beispielsweise die Na- und K-Salze von $C_8$-$C_{24}$-Carbonsäuren.

Weitere geeignete anionische Tenside sind lineare $C_8$-$C_{20}$-Alkylbenzolsulfonate ("LAS"), vorzugsweise lineare $C_9$-$C_{13}$-Alkylbenzolsulfonate und -Alkyltoluol-sulfonate.

Weiterhin eignen sich als anionische Tenside noch $C_8$-$C_{24}$-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbernsteinsäuren, Alkenylbernsteinsäuren oder deren Halbester oder Halbamide, Alkylsulfobemsteinsäuren oder deren Amide, Mono- und Diester von Sulfobernsteinsäuren, Acylsarkosinate, sulfatierte Alkylpolyglucoside, Alkylpolyglykolcarboxylate sowie Hydroxyalkylsarkosinate.

Die anionischen Tenside werden dem Wasch- und Reinigungsmittel vorzugsweise in Form von Salzen zugegeben. Geeignete Kationen in diesen Salzen sind Alkalimetallionen wie Natrium, Kalium und Lithium und Ammoniumsalze wie z.B. Hydroxyethylammonium-, Di(hydroxyethyl)ammonium- und Tri(hydroxyethyl)ammoniumsalze. Die anionischen Tenside liegen in den erfindungsgemäßen Waschmitteln vorzugsweise in einer Menge von bis zu 30 Gew.-%, beispielsweise von 0,1 bis 30 Gew.-%, vor allem 1 bis 25 Gew.-%, insbesondere 3 bis 20 Gew.-% vor. Werden $C_9$-$C_{20}$-linear-Alkylbenzolsulfonate (LAS) mitverwendet, kommen diese üblicherweise in einer Menge bis zu 15 Gew.-%, insbesondere bis zu 10 Gew.-%, zum Einsatz.

In den erfindungsgemäßen Reinigungsmitteln liegen die anionischen Tenside in einer Menge von bis zu 30 Gew.-%, vor allem bis zu 25 Gew.-%, insbesondere bis zu 15 Gew.-% vor. Werden $C_9$-$C_{20}$-linear-Alkyl-benzolsulfonate (LAS) mitverwendet, kommen diese üblicherweise in einer Menge bis zu 10 Gew.-%, insbesondere bis zu 8 Gew.-%, zum Einsatz.

Man kann einzelne anionische Tenside oder eine Kombination unterschiedlicher Aniontenside einsetzen. Es können anionische Tenside aus nur einer Klasse zum Einsatz gelangen, beispielsweise nur Fettalkoholsulfate oder nur Alkylbenzolsulfonate, man kann aber auch Tensid-Mischungen aus verschiedenen Klassen verwenden, z.B. eine Mischung aus Fettalkoholsulfaten und Alkylbenzolsulfonaten.

Ferner können die erfindungsgemäß einzusetzenden Tensid-Gemische der Formel (I) mit kationischen Tensiden, üblicherweise in einer Menge bis zu 25 Gew.-%, vorzugsweise 0,1 bis 15 Gew.-%, beispielsweise $C_8$-$C_{16}$-Dialkyldimethylammoniumhalogeniden, Dialkoxydimethylammoniumhalogeniden oder Imidazoliniumsalzen mit langkettigem Alkylrest; und/oder mit amphoteren Tensiden, üblicherweise in einer Menge bis zu 15 Gew.-%, vorzugsweise 0,1 bis 10 Gew.-%, beispielsweise Derivaten von sekundären oder tertiären Aminen wie z.B. $C_6$-$C_{18}$-Alkylbetainen oder $C_6$-$C_{15}$-Alkylsulfobetainen oder Aminoxiden wie Alkyldimethylaminoxiden kombiniert werden.

In der Regel werden die erfindungsgemäß einzusetzenden Tensid-Gemische der Formel (I) mit Buildern (Sequestrierungsmitteln) wie z.B. Polyphosphaten, Polycarboxilaten, Phosphonaten, Komplexbildnern, z.B. Methylglycindiessigsäure und deren Salze, Nitrilotriessigsäure und deren Salze, Ethylendiamintetraessigsäure und deren Salze sowie gegebenenfalls mit Co-Buildem kombiniert.

Einzelne zur Kombination mit den erfindungsgemäß einzusetzenden TensidGemischen der Formel (I) gut geeignete Buildersubstanzen seien im Folgenden aufgezählt:

Geeignete anorganische Builder sind vor allem kristalline oder amorphe Alumosilikate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in der US-A-4604224.

Als Builder geeignete kristalline Silicate sind beispielsweise Disilikate oder Schichtsilikate, z.B. $\delta$-$Na_2Si_2O_5$ oder $\beta$-$Na_2Si_2O_5$ (SKS 6 bzw. SKS 7). Die Silikate können in Form ihrer Alkalimetall-, Erdalkalimetall- oder Ammoniumsalze eingesetzt werden, vorzugsweise als Na-, Li- und Mg-Silikate. Amorphe Silikate wie beispielsweise Natriummetasilikat, welches eine polymere Struktur aufweist, oder amorphes Disilicat (Britesil® H 20 Hersteller: Akzo) sind ebenfalls verwendbar.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt. Übliche, als anorganische Builder eingesetzte Phosphate sind Alkali-orthophosphate, und/oder -Polyphosphate wie z.B. Pentanatriumtriphosphat. Die genannten Builder-Komponenten können einzeln oder in Mischungen untereinander eingesetzt werden.

Ferner ist es in vielen Fällen zweckmäßig den erfindungsgemäßen Wasch- und Reinigungsmitteln Co-Builder zuzufügen. Beispiele für geeignete Substanzen sind im Folgenden aufgelistet:

In einer bevorzugten Ausführungsform enthalten die erfindungsgemäßen Wasch- und Reinigungsmittel zusätzlich

zu den anorganischen Buildern 0,05 bis 20 Gew.-%, insbesondere 1 bis 10 Gew.-% organische Cobuilder in Form von niedermolekularen, oligomeren oder polymeren Carbonsäuren, insbesondere Polycarbonsäuren, oder Phosphonsäuren oder deren Salzen, insbesondere Na- oder K-salzen.

Als organische Cobuilder geeignete niedermolekulare Carbonsäuren oder Phosphonsäuren sind beispielsweise:

Phosphonsäuren wie z.B. 1-Hydroxyethan-1,1-diphosphonsäure, Amino-tris(methylenphosphonsäure), Ethylendiamin-tetra(methylenphosphonsäure), Hexamethylendiamintetra (methylenphosphonsäure) und Diethylentriaminpenta(methylenphosphonsäure); $C_4$-$C_{20}$-Di-, -Tri- und -Tetracarbonsäuren wie z.B. Bernsteinsäure, Propantricarbonsäure, Butantetracarbonsäure, Cyclopentantetra-carbonsäure und Alkyl- und Alkenylbernsteinsäuren mit $C_2$-$C_{16}$-Alkyl- bzw. -Alkenyl-Resten; $C_4$-$C_{20}$-Hydroxycarbonsäuren wie z.B. Äpfelsäure, Weinsäure, Gluconsäure, Glutarsäure, Citronensäure, Lactobionsäure und Saccharosemono-, -di- und -tricarbonsäure; Aminopolycarbonsäuren wie z.B. Nitrilotriessigsäure, β-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethytendiamin-dibemsteinsäure und N-(2-Hydroxyethyl)iminodiessigsäure, Methyl- und Ethylglycindiessigsäure.

Als organische Cobuilder geeignete oligomere oder polymere Carbonsäuren sind beispielsweise:

Oligomaleinsäuren, wie sie beispielsweise in EP-A 451508 und EP-A 396303 beschrieben sind; Co- und Terpolymere ungesättigter $C_4$-$C_8$-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können.

Als ungesättigte $C_4$- bis $C_8$-Dicarbonsäuren sind hierbei beispielsweise Maleinsäure. Fumarsäure, Itaconsäure und Citraconsäure geeignet. Bevorzugt wird Maleinsäure.

Die Gruppe (i) umfasst monoethylenisch ungesättigte $C_3$-$C_8$-Monocarbonsäuren wie z.B. Acrylsäure. Methacrylsäure, Crotonsäure und Vinylessigsäure. Bevorzugt werden aus der Gruppe (i) Acrylsäure und Methacrylsäure eingesetzt.

Die Gruppe (ii) umfasst monoethylenisch ungesättigte $C_2$-$C_{22}$-Olefine, Vinylalkylether mit $C_1$-$C_8$-Alkylgruppen, Styrol, Vinylester von $C_1$-$C_8$-Carbonsäuren, (Meth)acrylamid und Vinylpyrrolidon. Bevorzugt werden aus der Gruppe (ii) $C_2$-$C_6$-Olefine, Vinylalkylether mit $C_1$-$C_4$-Alkylgruppen, Vinylacetat und Vinylpropionat eingesetzt.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt.

Die Gruppe (iii) umfasst (Meth)acrylester von $C_1$-$C_8$-Alkoholen, (Meth)acrylnitril, (Meth)acrylamide von $C_1$-$C_8$-Aminen, N-Vinylformamid und N-Vinylimidazol.

Als organische Cobuilder eignen sich auch Homopolymere der monoethylenisch ungesättigten $C_3$-$C_8$-Monocarbonsäuren wie z.B. Acrylsäure, Methacrylsäure, Crotonsäure und Vinylessigsäure, insbesondere der Acrylsäure und Methacrylsäure, Copolymere von Dicarbonsäuren, wie z.B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000;

Terpolymere aus Maleinsäure, Acrylsäure und einem Vinylester einer $C_1$-$C_3$-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann; Copolymere von Maleinsäure mit $C_2$-$C_8$-Olefinen im Molverhältnis 40:60 bis 80:20, wobei Copolymere von Maleinsäure mit Ethylen, Propylen oder Isobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls als organische Cobuilder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z.B. sauer oder enzymatisch abgebaute Stärken, Inuline oder Zellulose, Eiweißhydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglykole mit Molmassen mit

bis zu $M_w$ = 5000 wie z. B. Polyethylenglykole, Ethylenoxid/Propylenoxid- bzw. Ethylenoxid/Butylenoxid bzw. Ethylenoxid/Propylenoxid/Butylenoxid-Blockcopolymere und alkoxylierte ein- oder mehrwertige $C_1$- bis $C_{22}$-Alkohole.(vgl. US-A-5756456)

Als organische Cobuilder geeignete Polyglyoxylsäuren sind beispielsweise beschrieben in EP-B-001004, US-A-5399286, DE-A-4106355 und EP-A-656914. Die Endgruppen der Polyglyoxylsäuren können unterschiedliche Strukturen aufweisen.

Als organische Cobuilder geeignete Polyamidocarbonsäuren und modifizierte Polyamidocarbonsäuren sind beispielsweise bekannt aus EP-A-454126, EP-B-511037, WO-A-94/01486 und EP-A-581452.

Als organische Cobuilder verwendet man insbesondere auch Polyasparaginsäuren oder Cokondensate der Asparaginsäure mit weiteren Aminosäuren, $C_4$-$C_{25}$-Mono- oder -Dicarbonsäuren und/oder $C_4$-$C_{25}$-Mono- oder -Diaminen. Besonders bevorzugt werden in phosphorhaltigen Säuren hergestellte, mit $C_6$-$C_{22}$-Mono- oder -Dicarbonsäuren bzw. mit $C_6$-$C_{22}$-Mono- oder -Diaminen modifizierte Polyasparaginsäuren eingesetzt.

Als organische Cobuilder eignen sich weiterhin Iminodibernsteinsäure, Oxydibemsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-$\alpha$-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Cobuilder verwendet werden.

In einer weiteren bevorzugten Ausführungsform enthalten die erfindungsgemäßen Wasch- und Reinigungsmittel zusätzlich, insbesondere zusätzlich zu den anorganischen Buildern, den anionischen Tensiden und/oder den nichtionischen Tensiden, 0,5 bis 20 Gew.-%, insbesondere 1 bis 10 Gew.-%, Glycin-N,N-diessigsäure-Derivate, wie sie in der WO 97/19159 beschrieben sind.

Häufig ist es auch zweckmäßig, den erfindungsgemäßen Wasch- und Reinigungsmitteln Bleichsysteme, bestehend aus Bleichmitteln, wie z.B. Perborat, Percarbonat und gegebenenfalls Bleichaktivatoren, wie z.B. Tetraacetylethylendiamin, + Bleichstabilisatoren zuzusetzen.

In diesen Fällen enthalten die erfindungsgemäßen Wasch- und Reinigungsmittel zusätzlich 0,5 bis 30 Gew.-%, insbesondere 5 bis 27 Gew.-%, vor allem 10 bis 23 Gew.-% Bleichmittel in Form von Percarbonsäuren, z.B. Diperoxododecandicarbonsäure, Phthalimidopercapronsäure oder Monoperoxophthalsäure oder -terephthalsäure, Addukten von Wasserstoffperoxid an anorganische Salze, z.B. NatriumperboratMonohydrat, Natriumperborat-Tetrahydrat, Natriumcarbonat-Perhydrat oder Natriumphosphat-Perhydrat, Addukten von Wasserstoffperoxid an organische Verbindungen, z.B. Hamstoff-Perhydrat, oder von anorganischen Peroxosalzen, z.B. Alkalimetallpersulfaten, oder -peroxodisulfaten, gegebenenfalls in Kombination mit 0 bis 15 Gew.-%, vorzugsweise 0,1 bis 15 Gew.-%, insbesondere 0,5 bis 8 Gew.-%, Bleichaktivatoren.

Als Bleichaktivatoren eignen sich:

- polyacylierte Zucker, z.B. Pentaacetylglucose;
- Acyloxybenzolsulfonsäuren und deren Alkali- und Erdalkaiimetallsalze, z.B. Natrium-p-nonanoyloxybenzolsulfonat oder Natrium-p-benzoyloxybenzolsulfonat;
- N,N-diacylierte und N,N,N',N'-tetraacylierte Amine, z.B. N,N,N',N'-Tetraacetylmethylendiamin und -ethylendiamin (TAED), N,N-Diaoetylanilin, N,N-Diacetyl-p-toluidin oder 1,3-diacyliente Hydantoine wie 1,3-Diacetyl-5,5-dimethylhydantoin;
- N-Alkyl-N-sulfonylcarbonamide, z.B. N-Methyl-N-mesylacetamid oder N-Methyl-N-mesylbenzamid;
- N-acylierte cydische Hydrazide, acylierte Triazole oder Urazole, z.B. Monoacetylmaleinsäurehydrazid;
- O,N,N-trisubstituierte Hydroxylamine, z.B. O-Benzoyl-N,N-succinylhydroxylamin, O-Acetyl-N,N-succinylhydroxylamin oder O,N,N-Triacetylhydroxylamin;
- N,N'-Diacylsulfurylamide, z.B. N,N'-Dimethyl-N,N'-diacetylsulfurylamid oder N,N'-Diethyl-N,N'-dipropionylsulfurylamid;
- acylierte Lactame wie beispielsweise Acetylcaprolactam, Octanoylcaprolactam, Benzoylcaprolactam oder Carbonylbiscaprolactam;
- Anthranilderivate wie z.B. 2-Methylanthranil oder 2-Phenylanthranil;
- Triacylcyanurate, z.B. Triacetylcyanurat oder Tribenzoylcyanurat;
- Oximester und Bisoximester wie z.B. O-Acetylacetonoxim oder Bisisopropyliminocarbonat;
- Carbonsäureanhydride, z.B. Essigsäureanhydrid, Benzoesäureanhydrid, m-Chlorbenzoesäureanhydrid oder Phthalsäureanhydrid;
- Enolester wie z.B. Isopropenylacetat;
- 1,3-Diacyl-4,5-diacyloxy-imidazoline, z.B. 1,3-Diacetyl-4,5-diacetoxyimidazolin;
- Tetraacetylglycoluril und Tetrapropionylglycoluril;
  diacylierte 2,5-Diketopiperazine, z.B. 1,4-Diacetyl-2,5-diketopiperazin;
- ammoniumsubstituierte Nitrile wie z.B. N-Methylmorpholiniumacetonitrilmethylsulfat;

- Acylierungsprodukte von Propylendiharnstoff und 2,2-Dimethyl-propylendihamstoff, z.B. Tetraacetylpropylendihamstoff;
- $\alpha$-Acyloxypolyacylmalonamide, z.B. $\alpha$-Acetoxy-N,N'-diacetylmalonamid;
- Diacyl-dioxohexahydro-1,3,5-triazine, z.B. 1,5-Diacetyl-2,4-dioxohexahydro-1,3,5-triazin;
- Benz-(4H)1,3-oxazin-4-one mit Alkylresten, z.B. Methyl, oder aromatischen Resten z:B. Phenyl, in der 2-Position.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quatemierte Imine und Sulfonimine, die beispielsweise beschrieben sind in US-A 5 360 569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Wasch- und Reinigungsmitteln höchstens in Mengen bis 1,5 Gew.-%, insbesondere bis 0,5 Gew.-%, im Falle von sehr aktiven Mangankomplexen in Mengen bis zu 0,1 Gew.-%, eingearbeitet.

Neben dem beschriebenen Bleichsystem aus Bleichmitteln, Bleichaktivatoren und gegebenenfalls Bleichkatalysatoren ist für die erfindungsgemäßen Wasch- und Reinigungsmittel auch die Verwendung von Systemen mit enzymatischer Peroxidfreisetzung oder von photoaktivierten Bleichsystemen möglich.

Für eine Reihe von Anwendungsfällen ist es zweckmäßig, wenn die erfindungsgemäßen Wasch- und Reinigungsmittel Enzyme enthalten. Vorzugsweise in Wasch- und Reinigungsmitteln eingesetzte Enzyme sind Proteasen, Amylasen, Lipasen und Cellulasen. Von den Enzymen werden vorzugsweise mengen von 0,1 bis 1,5 Gew.-%, insbesondere vorzugsweise 0,2 bis 1,0 Gew.-%, des konfektionierten Enzyms zugesetzt. Geeignete Proteasen sind z.B. Savinase und Esperase (Hersteller: Novo Nordisk). Eine geeignete Lipase ist z. B. Lipolase (Hersteller: Novo Nordisk). Eine geeignete Cellulase ist z.B. Celluzym (Hersteller: Novo Nordisk). Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann das erfindungsgemäße Wasch- und Reinigungsmittel noch Enzymstabilisatoren, z.B. Calciumpropionat, Natriumformiat oder Borsäuren oder deren Salze, und/oder Oxidationsverhinderer enthalten.

Die Bestandteile von Wasch- und Reinigungsmitteln sind dem Fachmann prinzipiell bekannt. Die obigen und die weiter unten folgenden Listen geeigneter Bestandteile geben nur einen exemplarischen Ausschnitt der bekannten geeigneten Bestandteile wieder.

Die erfindungsgemäßen Wasch- und Reinigungsmittel können neben den bisher genannten Hauptkomponenten noch folgende weitere übliche Zusätze in den hierfür üblichen Mengen enthalten:

Bekannte Dispergiermittel, wie z.B. Naphthalinsulfonsäurekondensate oder Polycarboxilate, pH-regulierende Verbindungen wie z.B. Alkalien bzw. Alkalispender (NaOH, KOH, Pentanatriummetasilikat) oder Säuren (Salzsäure, Phosphorsäure, Amidoschwefelsäure, Citronensäure) Puffersysteme, wie z.B. Acetat oder Phosphatpuffer,
Parfüm, Farbstoffe, Biozide, wie z.B. tsothiazolinone oder 2-Bromo-2-nitro-1,3-propandiol.
Solubilisatoren/Hydrotrope, wie z.B. Cumolsulfonate, Toluolsulfonate, kurzkettige Fettsäuren, Harnstoff, Alkohole oder Phosphorsäurealkyl/-arylester, Alkyl/Arylpolyglycolphosphorsäureester, Schaumregulatoren zur Stabilisierung oder Dämpfung des Schaums, Haut- und Korrosionsschutzmittel, desinfizierende Verbindungen oder Systeme, wie z.B. solche die Chlor oder unterchlorige Säure freisetzen wie z.B. Dichlorisocyanurat oder die Jod enthalten.

Die Waschmittel enthalten gegebenenfalls zusätzlich Schmutztragemittel, Soil release Agentien, wie z.B. Polyetherester, Inkrustationsinhibitoren, Ionenaustauscher, Vergrauungsinhibitoren, optische (fluoreszierende) Aufheller, Farbübertragungsinhibitoren wie z.B. Polyvinylpyrrolidon, Verdickungsmittel und Stell- und Konfektionierungsmittel, in Reinigungsmitteln können zusätzlich Lösemittel, wie z.B. kurzkettige Alkyloligoglykole wie Butylglykol, Butyldiglykol, Propylenglykolmonomethylether, Alkohole wie Ethanol, i-Propanol, aromatische Lösemittel wie Toluol, Xylol, N-Alkylpyrrolidone oder Alkylencarbonate, Verdicker, wie z.B. Polysaccharide, und/oder schwach vernetzte Polycarboxylate (beispielsweise Carbopol® der Firma Goodrich) feinteilige Abrasivkomponenten, wie z.B. Quarz- oder Marmormehl, Kreide, Diatomeenerde, Bimsstein oder auch Polierrot oder Schmirgel, enthalten sein.

Die Waschmittel liegen gewöhnlich, aber nicht ausschließlich, in fester, pulverförmiger Form vor, und enthalten dann in der Regel zusätzlich übliche Stellmittel, die ihnen eine gute Rieselfähigkeit. Dosierbarkeit und Löslichkeit verleihen und die das Zusammenbacken und Stauben verhüten, wie z.B. Natriumsulfat oder Magnesiumsulfat. Die pulverförmigen Waschmittel haben in der herkömmlichen Form ein durchschnittliches Schüttgewicht von ca. 450 g/l. Kompakt- oder Ultra-Kompaktwaschmittel sowie Extrudate weisen ein Schüttgewicht > 600 g/l auf. Diese gewinnen immer mehr an Bedeutung.

Sofern sie in flüssiger Form eingesetzt werden sollen, können sie als wässrige Mikroemulsionen, Emulsionen oder Lösungen vorliegen. In flüssigen Waschmitteln können zusätzlich Lösungsmittel wie z.B. Ethanol, i-Propanol, 1,2-Propylenglykol, oder Butylglykol verwendet werden.

Bei gelförmigen erfindungsgemäßen Waschmitteln können zusätzlich Verdicker, wie z.B. Polysaccharide und/oder schwach vernetzte Polycarboxylate (beispielsweise Carbopol® der Fa. Goodrich) eingesetzt werden.

Bei tablettenförmigen Waschmitteln werden zusätzlich Tablettierhilfsmittel wie z.B. Polyethylenglykole mit Molmassen > 1000 g/mol, Polymerdispersionen, und Tablettensprengmittel wie z.B. Cellulosederivate, vernetztes Polyvinylpyrrolidon, vernetzte Polyacrylate oder Kombinationen aus Säuren, z.B. Citronensäure + Natriumbicarbonat, um nur einige zu nennen, benötigt.

Die Reinigungsmittel sind gewöhnlich, aber nicht ausschließlich, wässrig und liegen in der Form von Mikroemulsionen, Emulsionen oder Lösungen vor.

Sollten sie in fester, pulverförmiger Form vorliegen, können zusätzlich übliche Stellmittel, die ihnen eine gute Rieselfähigkeit, Dosierbarkeit und Löslichkeit verleihen und/oder die das Zusammenbacken und Stauben verhüten, wie z.B. Natriumsulfat oder Magnesiumsulfat eingesetzt werden.

Bei tablettenförmigen Reinigern werden zusätzlich Tablettierhilfsmittel wie z.B. Polyethylenglykole mit Molmassen > 1000 g/mol, Polymerdispersionen, und Tablettensprengmittel wie z.B. Cellulosederivate, vernetztes Polyvinylpyrrolidon, vernetzte Polyacrylate oder Kombinationen aus Säuren, z.B. Citronensäure + Natriumbicarbonat, um nur einige zu nennen, benötigt.

Die vorliegende Erfindung wird anhand der nachfolgenden Beispiele näher erläutert.

## Beispiele

Beispiel 1: Tridecylpentacosaoxyethylenalykol-C8-C18-Kokosfettsäureester (hydriert) (A)

a) Herstellung des Alkylalkoxylats:

Tridekanol N (160 g, 0.8 mol; Hersteller BASF) wird mit gepulvertem KOH (2 g, 0.036 mol)in einem 21 Druckautoklaven der Firma Mettler versetzt und 1 h bei 95°C und 20 mbar entwässert. Anschließend wird zweimal mit Stickstoff inertisiert und auf 100°C erwärmt. Innerhalb von 8 h wird bis zu einem maximalen Druck von 6 bar Ethylenoxid (880 g, 20 mol) zudosiert und nach beendeter Zugabe noch 3 h nachgerührt. Abschließend wird die Verbindung mit Ambosol (3 Gewichtsprozent) versetzt und fitriert. Man erhält Tridecylpentacosaoxyethylenglykol (1040g; OH-Zahl 45 mg KOH/g, Theorie 43.2 mg KOH/g) als weißen Feststoff.

b) Veresterung:

Tridecylpentacosaoxyethylenglykol (149.5 g, 0.12 mol) wird mit hydrierter C8-C18-Kokosfettsäure (24.8 g, 0.12 mol; Edenor HK 818, Firma Cognis), para-Toluolsulfonsäure (1.1 g, 0.06 mol) und Toluol (50 ml) versetzt und bei 140 °C am

Wasserabscheider für 10 h gekocht. Man erhält 170 g Feststoff (Schmp.: 33°C) mit einem Veresterungsgrad von >95% $^1$H-NMR

*Typische Zusammensetzung von Edenor HK 18 lt. Hersteller:

| Kettenverteilung | Spezifizierte Grenzwerte | Typische Werte |
|---|---|---|
| C6 | 0-1 | Spuren |
| C8 | 2-10 | 6 |
| C 10 | 4-8 | 6 |
| C12 | 45-55 | 49 |
| C14 | 17-21 | 19 |
| C16 | 7-13 | 10 |
| C18 | 7-14 | 10 |
| >C18 | 0-0.5 | Spuren |

Beispiel 2: 2-Propylheptylcosaoxyethylenglykol-Dekansäureester (B)

a) Herstellung des Alkylalkoxylats:

2-Propylheptanol (158.3 g, 1.0 mol; Hersteller BASF) wird mit gepulvertem KOH (2.1 g, 0.038 mol) in einem 21 Druckautoklaven der Firma Mettler versetzt und 1 h bei 95°C und 20 mbar entwässert. Anschließend wird zweimal

mit Stickstoff inertisiert und auf 100°C erwärmt. Innerhalb von 8 h wird bis zu einem maximalen Druck von 6 bar Ethylenoxid (880 g, 20 mol) zudosiert und nach beendeter Zugabe noch 3 h nachgerührt. Abschließend wird die Verbindung mit Ambosol (3 Gewichtsprozent) versetzt und filtriert. Man erhält 2-Propylheptylcosaoxyethylenglykol (1035 g; OH-Zahl 54 mg KOH/g, Theorie 55 mg KOH/g) als weißen Feststoff.

b) Veresterung:

2-Propylheptylcosaoxyethylenglykol (124.6 g, 0.12 mol) wird mit Dekansäure (20.7 g, 0.12 mol), para-Toluolsulfonsäure (1.1 g, 0.06 mol) und Toluol (50 ml) versetzt und bei 140 °C am Wasserabscheider für 10 h gekocht. Man erhält 140 g Feststoff (Schmp.: 30°C) mit einem Veresterungsgrad von 93% ($^1$H-NMR).

<u>Beispiel 3: 2-Propylheptyldecaoxyethylenglykol-Dekansäureester (C, Stand der Technik)</u>

Lutensol® XP 100 (168.8 g, 0.4 mol; Firma BASF) wird mit Dekansäure (68.8 g, 0.4 mol), para-Toluolsulfonsäure (1.1 g, 0.06 mol) und Toluol (50 ml) versetzt und bei 140 °C am Wasserabscheider für 10 h gekocht. Man erhält 230 g flüssiger Substanz mit einem Veresterungsgrad von 80% ($^1$H-NMR)

<u>Beispiel 4: 2-Propylheptylhexaoxyethylenglykol-Dekansäureester (D, Stand der Technik)</u>

Lutensol® XP 60 (101.3 g, 0.24 mol; Firma BASF) wird mit Dekansäure (49.6 g, 0.24 mol), para-Toluolsulfonsäure (2.2 g, 0.12 mol) und Toluol (50 ml) versetzt und bei 140°C am Wasserabscheider für 10 h gekocht. Man erhält 140 g flüssiger Substanz mit einem Veresterungsgrad von 90% ($^1$H-NMR)

<u>Beispiel 5: 2-Propylheptyloxypropylencosaoxyethylenglykol-Dekansäureester (E)</u>

a) Herstellung des Alkylalkoxylats:

2-Propylheptanol (395.8 g, 2.5 mol; Hersteller BASF) wird mit gepulvertem KOH (11 g, 0.20 mol) in einem 3,51 Druckautoklaven der Firma Mettler versetzt und 1 h bei 95°C und 20 mbar entwässert. Anschließend wird zweimal mit Stickstoff inertisiert und auf 120°C erwärmt. Man dosiert innerhalb von 1 h bis zu einem maximalen Druck von 2 bar Propylenoxid (145 g, 2 mol) zu und lässt 2 h bei Druckkonstanz nachrühren. Anschließend wird bei 120°C innerhalb von 8 h bis zu einem maximalen Druck von 6 bar Ethylenoxid (880 g, 50 mol) zudosiert und nach beendeter Zugabe noch 3 h nachgerührt. Abschließend wird die Verbindung mit Ambosol (3 Gewichtsprozent) versetzt und filtriert. Man erhält 2-Propylheptyloxypropylencosaoxyethylenglykol (2744 g; OH-Zahl 52 mg KOH/g, Theorie 51 mg KOH/g) als weißen Feststoff,

b) Veresterung:

2-Propylheptyloxypropylencosaoxyethylenglykol (165 g, 0.15 mol) wird mit Dekansäure (25.8 g, 0.15 mol), para-Toluolsulfonsäure (1.4 g, 0.075 mol) und Toluol (50 ml) versetzt und bei 140 °C am Wasserabscheider für 10 h gekocht. Man erhält 189 g Feststoff mit einem Veresterungsgrad von 82% ($^1$H-NMR)

<u>Beispiel 6: 2-Propylheptylcosaoxyethylenoxypropylenglykol-Dekansäureester (F)</u>

a) Herstellung des Alkylalkoxylats:

2-Propylheptanol (158.3 g, 1.0 mol; Hersteller BASF) wird mit gepulvertem KOH (4.4 g, 0.078 mol) in einem 2l Druckautoklaven der Firma Mettler versetzt und 1 h bei 95°C und 20 mbar entwässert. Anschließend wird zweimal mit Stickstoff inertisiert und auf 120 °C erwärmt. Innerhalb von 8 h wird bis zu einem maximalen Druck von 8 bar Ethylenoxid (880 g, 20 mol) zudosiert und nach beendeter Zugabe noch 6 h nachgerührt. Anschließend wird der Reaktor auf Normaldruck entspannt und innerhalb von 2 h bis zu einem Druck von 7 bar Propylenoxid (58 g, 1 mol) bei 120 °C zudosiert. Abschließend wird die Verbindung mit Ambosol (3 Gewichtsprozent) versetzt und filtriert. Man erhält 2-Propylheptylcosaoxyethylenglykol (1030 g; OH-Zahl 54 mg KOH/g, Theorie 51 mg KOH/g) als weißen Feststoff.

b) Veresterung:

2-Propylheptyloosaoxyethylenoxypropylenglykol (124.7 g, 0.12 mol) wird mit Dekansäure (20.6 g, 0.12 mol), para-Toluolsulfonsäure (1.1 g, 0.06 mol) und Toluol (50 ml) versetzt und bei 140 °C am Wasserabscheider für 10 h

gekocht. Man erhält 142 g Feststoff mit einem Veresterungsgrad von 90% ($^1$H-NMR)

Beispiel 7: Tridecylheptacosaoxyethylenmonopropylenglykol-C6-C14-Carbonsäureester (G)

a) Herstellung des Alkylalkoxylats:

Tridekanol N (140 g, 0.7 mol; Hersteller BASF) wird mit 50%iger wässriger KOH (4.4 g wässrige Lösung, 0.039 mol) in einem 21 Druckautoklaven der Firma Mettler versetzt und 1 h bei 95°C und 20 mbar entwässert. Anschließend wird zweimal mit Stickstoff inertisiert und auf 120°C erwärmt. Innerhalb von 12 h wird bis zu einem maximalen Druck von 6 bar Ethylenoxid (832 g, 18.9 mol) zudosiert und nach beendeter Zugabe noch 2 h nachgerührt. Anschließend wird der Reaktor auf Normaldruck entspannt und innerhalb von 2 h bis zu einem Druck von 7 bar Propylenoxid (40.6 g, 0.7 mol) bei 120 °C zudosiert und 2 h nachgerührt. Man erhält Tridecylheptacosaoxyethylenmonopmpylenglykol (1020g; OH-Zahl 40 mg KOH/g, Theorie 39 mg KOH/g) als weißen Feststoff.

b) Veresterung:

Tridecylheptacosaoxyethylenmonopropylenglykol (100 g, 0.071 mol) wird mit C6C10-Methylester (6.0 g, 0.036 mol; Edenor ME C6-10, Firma Cognis*) und C12C14-Methylester (7.8 g, 0.036 mol; Edenor ME C1270, Firma Cognis*) und bei 160 °C unter N2-Strom für 4 h gekocht. Man erhält 107 g beigen Feststoff (Schmp.: 33-35°C) mit einem Veresterungsgrad von 75% ($^1$H-NMR). Dieser lässt sich durch Zugabe von $H_2O_2$ (3.3 g einer 30%igen Lösung in Wasser) und 20 min Rühren bei 60°C sowie Neutralisation mit Essigsäure (0.23 g, 0.004 mol) in einen weißen Feststoff überführen.

*Typische Zusammensetzung von Edenor ME C6-10 laut Hersteller:

| Kettenverteilung | Spezifizierte Grenzwerte |
|---|---|
| C6 | 3-8 |
| C8 | 40-65 |
| C10 | 35-50 |
| C12 | 0-5 |

*Typische Zusammensetzung von Edenor ME C1270 laut Hersteller:

| Kettenverteilung | Spezifizierte Grenzwerte |
|---|---|
| C10 | <2 |
| C12 | 70-75 |
| C14 | 22-30 |
| C16 | <2 |
| C18 | <0,5 |

Beispiel 8: Schaumvolumen in der Geschirrspülmaschine

Das Schaumvolumen in der Geschirrspülmaschine wird überprüft. Dabei werden 10 ml Hühnerei, 19 g eines Basisgeschirrspülmittels (Hauptbestandteile 48% Natriummetasilikat x $5H_2O$, 45% Natriumtriphosphat, 5% Natriumcarbonat) und 1 g des Tensids in die Spülmaschine gegeben. Bei unterschiedlichen Temperaturen wird dann die Zahl der Umdrehungen des Sprüharms gemessen. Bei hohem Schaumniveau wird der Sprüharm abgebremst, bei niedrigem kann er mit höchstmöglicher Geschwindigkeit (ca. 150 U/min) arbeiten.

Verschiedene Tenside wurden in dieser Anwendung geprüft.

| Name | Tensidhauptkomponente |
|---|---|
| A | $C_{13}$-Alkohol-25 EO + hydrierte Kokosfettsäure (Cognis: Edenor HK 818) |
| B | 2-PH-20 EO + Dekansäure |
| C (Stand der Technik) | 2-PH-10 EO + Dekansäure |
| D (Stand der Technik | 2-PH-6 EO + Dekansäure |
| E | 2-PH-1 PO-20 EO + Dekansäure |
| F | 2-PH-20 EO-1 PO + Dekansäure |
| G | $C_{13}$-Alkohol-27 EO-1 PO + $C_6C_{14}$-Methylester |

Die Umdrehungsgeschwindigkeit wurde bei 30, 40, 50, 60°C gemessen. In folgender Tabelle sind die Rotorgeschwindigkeiten in U/Min bei verschiedenen Temperaturen aufgelistet.

| Temperatur | A | B | C | D | E | F | G |
|---|---|---|---|---|---|---|---|
| 30°C | 119 | 122 | 128 | 131 | 113 | 123 | 117 |
| 40°C | 127 | 126 | 131 | 129 | 120 | 127 | 113 |
| 50°C | 126 | 129 | 129 | 125 | 128 | 129 | 111 |
| 60°C | 128 | 130 | 127 | 112 | 128 | 129 | 125 |

Beispiel 9: Kontaktwinkel auf harten Oberflächen

In folgender Tabelle sind die Kontaktwinkel von wässrigen Tensidlösungen (0,2 g/l) nach 0,1s, 1s und 10s in Grad bei 40°C auf verschiedenen harten Oberflächen wiedergegeben. Je kleiner der Winkel ist, desto ausgeprägter ist die Benetzung. Es wurden verschiedene Tenside getestet.

| Kontakt winkel | A | B | C | D | E | F | G | Wasser |
|---|---|---|---|---|---|---|---|---|
| Glas | 39/35/23 | 43/40/29 | 44/41/33 | 36/34/28 | 41/37/24 | 35/32/20 | 43/36/29 | 45/42/41 |
| Stahl | 62/62/48 | 65/61/50 | 68/67/61 | 70/70/68 | 61/60/46 | 63/62/49 | 51/44/32 | 82/78/78 |
| Polyethylen | 65/65/52 | 71/68/61 | 73/72/71 | 75/74/74 | 72/69/58 | 68/67/57 | 58/53/41 | 94/95/94 |

Beispiel 10 Formulierbarkeit

In folgender Tabelle ist die Formulierbarkeit (1 g Tensid auf 49 g Reinigerlösung) in zwei typischen alkalischen Reinigerformulierungen wiedergegeben. Beurteilt wird, ob sich das Tensid in den Reinigerformulierungen klar löst. Sind die Formulierungen klar, so wird noch der Trübungspunkt bestimmt (Verhalten bei 20°C/Trübungspunkt in °C).
Es wurden verschiedene Tenside getestet.

| Reiniger | A | B | C | D | E | F | G |
|---|---|---|---|---|---|---|---|
| pH 7,5 | klar/64 | klar/59 | trüb/- | trüb/- | klar/64 | klar/66 | nicht gemessen |
| pH 9,2 | klar/65 | klar/61 | trüb/- | trüb/- | klar/66 | klar/67 | klar/59 |

Beispiel 11 Stabilitätstests über den Anwendungsbereich

Der Versuch erfolgt analog zu Beispiel 8. Die Messung der Umdrehungszahlen bei Maximaltemperatur (60°C - 69°C) erfolgt über einen Zeitraum von 2h.

| Zeit [min] | 10 | 20 | 30 | 40 | 50 | 60 | 70 | 80 | 90 | 100 | 110 | 120 |
|---|---|---|---|---|---|---|---|---|---|---|---|---|
| E | 128 | 126 | 126 | 125 | 125 | 123 | 123 | 121 | 117 | 113 | 103 | 91 |
| F | 130 | 128 | 128 | 127 | 128 | 127 | 127 | 127 | 125 | 125 | 125 | 123 |

**Patentansprüche**

**1.** Schaumarme Tensid-Mischung enthaltend Verbindungen der allgemeinen Formel (I)

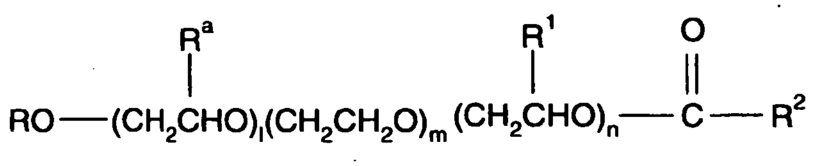

(I)

wobei

R ein verzweigter oder unverzweigter Alkylrest mit 8 bis 16 Kohlenstoffatomen ist;
$R^a$, $R^1$ unabhängig voneinander Wasserstoff oder ein verzweigter oder unverzweigter Alkylrest mit 1 bis 5 Kohlenstoffatomen sind;
$R^2$ ein unverzweigter Alkylrest mit 5 bis 17 Kohlenstoffatomen ist;
l, n unabhängig voneinander eine Zahl von 1 bis 5 sind und
m eine Zahl von 13 bis 35 ist.

**2.** Tensid-Mischung nach Anspruch 1, **dadurch gekennzeichnet, dass** Verbindungen gemäß der Formel (II)

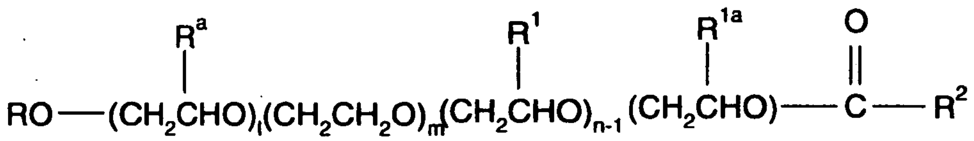

(II)

enthalten sind, wobei $R^{1a}$ ein verzweigter oder unverzweigter Alkylrest mit 1 bis 5 Kohlenstoffatomen ist und R, $R^a$, $R^1$, $R^2$, l, m und n die Bedeutung wie in Anspruch 1 haben.

**3.** Tensid-Mischung nach Anspruch 1 oder 2, **dadurch gekennzeichnet, dass** mindestens eine der folgenden Angaben erfüllt ist:

a) R ist ein verzweigter Alkylrest mit 10 bis 13 Kohlenstoffatomen;
b) $R^a$, $R^1$ sind unabhängig voneinander Wasserstoff, Methyl oder Ethyl;
c) $R^{1a}$ ist Methyl oder Ethyl;
d) $R^2$ ist ein verzweigter oder unverzweigter Alkylrest mit 5 bis 13 Kohlenstoffatomen;
e) n = 1 und l = 5;
f) m ist eine Zahl von 17 bis 27.

**4.** Tensid-Mischung nach einem der Ansprüche 1 bis 3, **dadurch gekennzeichnet, dass** das mittlere Molekulargewicht von 950 g/mol bis 2300 g/mol beträgt.

**5.** Tensid-Mischung nach einem der Ansprüche 1 bis 4, **dadurch gekennzeichnet, dass** der Beginn des Schmelzbereiches der Mischung über 25 °C liegt.

**6.** Tensid-Mischung nach einem der Ansprüche 1 bis 5, **dadurch gekennzeichnet, dass** mehr als 50% der Verbindungen der Tensid-Mischung Verbindungen der Formel (II) sind oder Verbindungen der Formel (I), in denen min-

destens ein $R^1$ kein Wasserstoff ist, sind.

7. Verfahren zur Herstellung von Tensid-Mischungen nach einem der Ansprüche 1 bis 6, die Schritte enthaltend:

a) Umsetzung eines Alkohols der Formel ROH mit einem Epoxid der Formel

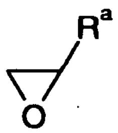

und anschließend mit Ethylenoxid;
b) Umsetzung des Produktes aus Schritt a) mit einem Epoxid der Formel

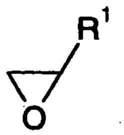

und optional mit einem Epoxid der Formel

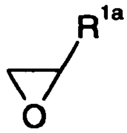

c) Umsetzung des gebildeten Produkts aus Schritt b) mit einer Carbonsäure $R^2$-COOH oder eines Methylesters der Formel $R^2$-$COOCH_3$, wobei $R^1$, $R^{1a}$ und $R^2$ die Bedeutung wie in Anspruch 1 oder 2 aufweisen.

8. Verfahren nach Anspruch 7, **dadurch gekennzeichnet, dass** mindestens eine der folgenden Bedingungen erfüllt ist:

a) die Umsetzung in Schritt a) und b) findet unter wasserfreier Basenkatalyse statt;
b) die Umsetzung in Schritt a) und b) findet in einem Temperaturbereich von 50 bis 200 °C statt;
c) die Umsetzung in Schritt c) findet säurebasekatalysiert im Fall von $R^2$-COOH oder basenkatalysiert im Fall von $R^2$-$COOCH_3$ statt;
d) die Umsetzung in Schritt c) findet in einem Temperaturbereich von 80 bis 200 °C statt;
e) die Umsetzung in Schritt c) findet unter kontinuierlicher Entfernung des Reaktionswassers bzw. Methanols statt.

9. Wasch- oder Reinigungsmittel-Formulierung enthaltend eine Tensid-Mischung nach einem der Ansprüche 1 bis 6.

10. Verwendung von einer Tensid-Mischung nach einem der Ansprüche 1 bis 6 in Wasch- und Reinigungsmittelformulierungen.

## Claims

1. A low-foam surfactant mixture comprising compounds of the general formula (I)

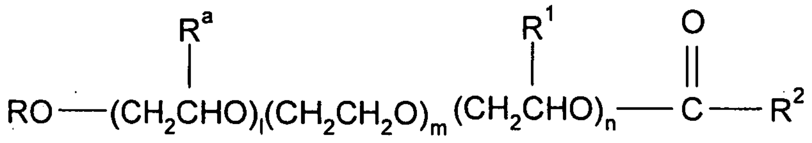 (I)

where

R is a branched or unbranched alkyl radical having 8 to 16 carbon atoms;
$R^a$, $R^1$ independently of one another, are hydrogen or a branched or unbranched alkyl radical having 1 to 5 carbon atoms;
$R^2$ is an unbranched alkyl radical having 5 to 17 carbon atoms;
l, n independently of one another, are a number from 1 to 5 and
m is a number from 13 to 35.

**2.** The surfactant mixture according to claim 1, wherein compounds according to the formula (II)

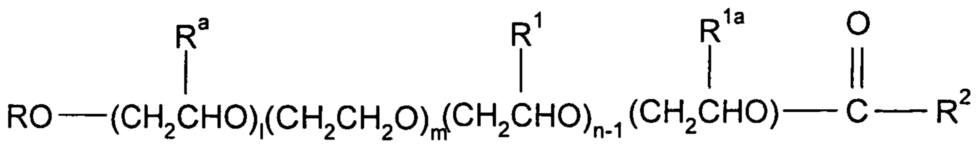 (II)

are present, where $R^{1a}$ is a branched or unbranched alkyl radical having 1 to 5 carbon atoms and R, $R^a$, $R^1$, $R^2$, l, m and n have the meaning as in claim 1.

**3.** The surfactant mixture according to claim 1 or 2, wherein at least one of the following requirements is satisfied:

a) R is a branched alkyl radical having 10 to 13 carbon atoms;
b) $R^a$, $R^1$, independently of one another, are hydrogen, methyl or ethyl;
c) $R^{1a}$ is methyl or ethyl;
d) $R^2$ is a branched or unbranched alkyl radical having 5 to 13 carbon atoms;
e) n = 1 and l = 5;
f) m is a number from 17 to 27.

**4.** The surfactant mixture according to one of claims 1 to 3, wherein the average molecular weight is from 950 g/mol to 2300 g/mol.

**5.** The surfactant mixture according to one of claims 1 to 4, wherein the start of the melting range of the mixture is above 25°C.

**6.** The surfactant mixture according to one of claims 1 to 5, wherein more than 50% of the compounds of the surfactant mixture are compounds of the formula (II) or compounds of the formula (I) in which at least one $R^1$ is not hydrogen.

**7.** A method of producing surfactant mixtures according to one of claims 1 to 6, the steps comprising:

a) reaction of an alcohol of the formula ROH with an epoxide of the formula

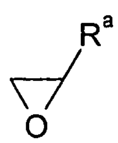

and then with ethylene oxide;

b) reaction of the product from step a) with an epoxide of the formula

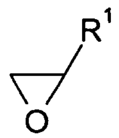

and optionally with an epoxide of the formula

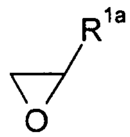

c) reaction of the product formed from step b) with a carboxylic acid $R^2$-COOH or a methyl ester of the formula $R^2$-$COOCH_3$, where $R^1$, $R^{1a}$ and $R^2$ have the meaning as in claim 1 or 2.

**8.** The method according to claim 7, wherein at least one of the following conditions is satisfied:

a) the reaction in step a) and b) takes place under anhydrous base catalysis;
b) the reaction in step a) and b) takes place in a temperature range from 50 to 200°C;
c) the reaction in step c) takes place under acid-base catalysis in the case of $R^2$-COOH or base catalysis in the case of $R^2$-$COOCH_3$;
d) the reaction in step c) takes place in a temperature range from 80 to 200°C;
e) the reaction in step c) takes place with the continuous removal of the water of reaction or methanol.

**9.** A detergent or cleaner formulation comprising a surfactant mixture according to one of claims 1 to 6.

**10.** The use of a surfactant mixture according to one of claims 1 to 6 in detergent and cleaner formulations.

## Revendications

**1.** Mélange tensioactif pauvre en mousse contenant des composés de formule générale (I) :

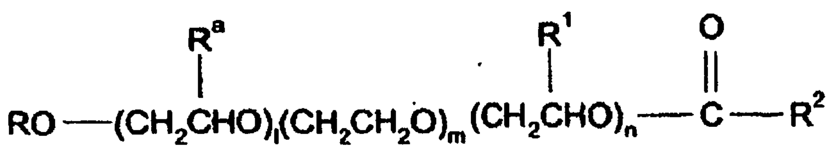

**(I)**

dans laquelle :

R est un radical alkyle ramifié ou non ramifié avec 8 à 16 atomes de carbone ;
$R^a$, $R^1$ sont indépendamment l'un de l'autre de l'hydrogène ou un radical alkyle ramifié ou non ramifié avec 1 à 5 atomes de carbone ;
$R^2$ est un radical alkyle non ramifié avec 5 à 17 atomes de carbone ;
1, n sont indépendamment l'un de l'autre des nombres de 1 à 5 ;
m est un nombre de 13 à 35.

**2.** Mélange tensioactif selon la revendication 1, **caractérisé en ce que** sont contenus des composés de formule (II) :

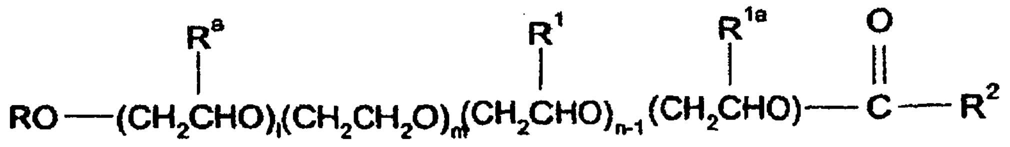 (II)

$R^{1a}$ étant un radical alkyle ramifié ou non ramifié avec 1 à 5 atomes de carbone et R, $R^a$, $R^1$, $R^2$, 1, m et n ont la même signification que dans la revendication 1.

3. Mélange tensioactif selon la revendication 1 ou 2, **caractérisé en ce qu'**au moins une des données suivantes est vérifiée :

a) R est un radical alkyle ramifié avec 10 à 13 atomes de carbone ;
b) $R^a$, $R^1$ sont indépendamment l'un de l'autre de l'hydrogène, du méthyle ou de l'éthyle ;
c) $R^{1a}$ est du méthyle ou de l'éthyle ;
d) $R^2$ est un radical alkyle ramifié ou non ramifié avec 5 à 13 atomes de carbone ;
e) n = 1 et l = 5 ;
f) m est un nombre de 17 à 27.

4. Mélange tensioactif selon l'une quelconque des revendications 1 à 3, **caractérisé en ce que** le poids moléculaire moyen est de 950 g/mole à 2300 g/mole.

5. Mélange tensioactif selon l'une quelconque des revendications 1 à 4, **caractérisé en ce que** le début de la plage de fusion du mélange se situe au-dessus de 25 °C.

6. Mélange tensioactif selon l'une quelconque des revendications 1 à 5, **caractérisé en ce que** plus de 50 % des composés du mélange tensioactif sont des composés de formule (II) ou sont des composés de formule (I), dans lesquelles au moins un radical $R^1$ n'est pas de l'hydrogène.

7. Procédé de préparation de mélanges tensioactifs selon l'une quelconque des revendications 1 à 6, comprenant les étapes consistant à :

a) amener à réagir un alcool de formule ROH avec un époxyde de formule :

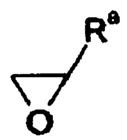

puis ensuite avec de l'oxyde d'éthylène ;
b) amener à réagir le produit de l'étape a) avec un époxyde de formule :

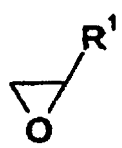

et optionnellement avec un époxyde de formule :

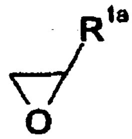

c) amener à réagir le produit formé dans l'étape b) avec un acide carboxylique $R^2$-COOH ou un ester de méthyle de formule $R^2$-$COOCH_3$, $R^1$, $R^{1a}$ et $R^2$ présentant la même signification que dans la revendication 1 ou 2.

**8.** Procédé selon la revendication 7, **caractérisé en ce qu'**au moins une des conditions suivantes est remplie :

a) la réaction des étapes a) et b) est mise en oeuvre sous catalyse basique anhydre ;
b) la réaction dans les étapes a) et b) a lieu dans une plage de températures de 50 à 200 °C ;
c) la réaction dans l'étape c) est mise en oeuvre par catalyse acide basique dans le cas de $R^2$-COOH ou par catalyse basique dans le cas de $R^2$-$COOCH_3$ ;
d) la réaction dans l'étape c) a lieu dans une plage de températures allant de 80 à 200 °C ;
e) la réaction dans l'étape c) a lieu avec élimination en continu de l'eau réactionnelle ou encore du méthanol.

**9.** Formulation de détergent ou de produit de nettoyage contenant un mélange tensioactif selon l'une quelconque des revendications 1 à 6.

**10.** Utilisation d'un mélange tensioactif selon l'une quelconque des revendications 1 à 6 dans des formulations de détergent ou de produit de nettoyage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1243312 A
- DE 2544707 A
- EP 035702 A
- WO 9403251 A
- WO 9511225 A
- US 4604224 A
- EP 451508 A
- EP 396303 A
- US 3887806 A
- DE 4313909 A
- US 5227446 A
- DE 4415623 A
- US 5756456 A
- EP 001004 B
- US 5399286 A
- DE 4106355 A
- EP 656914 A
- EP 454126 A
- EP 511037 B
- WO 9401486 A
- EP 581452 A
- WO 9719159 A
- US 5360569 A
- EP 453003 A
- WO 9421777 A


### In der Beschreibung aufgeführte Nicht-Patentliteratur


- Physikalische Eigenschaften und Wirkungen der Tenside. **H.-D. DÖRFER.** Grenzflächen und kolloid-disperse Systeme. Springer Verlag, 2002
- **HERMANN G. HAUTHAL ; G. WAGNER.** Reinigungs- und Pflegemittel im Haushalt. Verlag für chemische Industrie, 2003, 161-184